(19) Europäisches Patentamt
European Patent Office
Office européen des brevets

(11) **EP 4 411 745 A1**

(12) **EUROPEAN PATENT APPLICATION**

(43) Date of publication:
**07.08.2024 Bulletin 2024/32**

(21) Application number: **23154428.9**

(22) Date of filing: **01.02.2023**

(51) International Patent Classification (IPC):
**G16H 20/10** (2018.01)          **G16H 40/63** (2018.01)
**G16H 40/67** (2018.01)

(52) Cooperative Patent Classification (CPC):
**G16H 20/10; G16H 40/63; G16H 40/67**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Pari Medical Holding GmbH
82319 Starnberg (DE)**

(72) Inventors:
• **Fuchs, Carola
82061 Neuried (DE)**
• **Finke, Matthias
82152 Planegg (DE)**
• **Fiebig, David Vincent
81545 München (DE)**

(74) Representative: **Hoffmann Eitle
Patent- und Rechtsanwälte PartmbB
Arabellastraße 30
81925 München (DE)**

(54) **MEDICAL EVALUATION METHOD, AEROSOL NEBULIZER AND MEDICAL EVALUATION SYSTEM**

(57)     Provided is a medical evaluation method for evaluating a validity of a nebulizing session prescribed by a therapy protocol when using an aerosol nebulizer (30) comprising an aerosol generator (31) for nebulizing a liquid medication, the method comprising: obtaining (S100) nebulizing data indicating a first nebulizing operation and a second nebulizing operation of the aerosol nebulizer, each of the first and second nebulizing operation having an individual nebulizing duration at an individual nebulizing time and a nebulizing status code; deciding (S200) whether to perform clustering, based on the individual nebulizing time, the nebulizing status code and/or the individual nebulizing duration of the first and/or the second nebulizing operation and/or the liquid medication to be nebulized by the first and second nebulizing operation; and if it is decided to perform clustering, the method further comprising: clustering (S320) the first and second nebulizing operation into the nebulizing session; determining (S320) a total nebulizing duration for the nebulizing session based on the individual nebulizing duration of the first and second nebulizing operation clustered into the nebulizing session; and evaluating (S330) the validity of the nebulizing session based on a prescribed nebulizing duration specific to the therapy protocol and the total nebulizing duration determined for the nebulizing session.

Fig. 3

EP 4 411 745 A1

**Description**

TECHNICAL FIELD

The present invention relates to a medical evaluation method, an aerosol nebulizer and a medical evaluation system, and in particular to medical evaluation method, an aerosol nebulizer and a medical evaluation system for evaluating a validity of a nebulizing session prescribed by a therapy protocol when using the aerosol nebulizer.

BACKGROUND

To perform an adherence evaluation, nebulizing data associated with an inhalation process may need to be transmitted to a medical evaluation device. However, the quality and correctness of the nebulizing data determines the quality and correctness of the adherence evaluation. To transmit nebulizing data to the medical evaluation device for performing the adherence evaluation, a continuous communication connection to the medical evaluation device may be required. Interruptions of or disturbances on the communication connection may result in an incorrect adherence evaluation. Further, there are occurrences, causing the inhalation process to be interrupted. Such interruptions may be based on technical reasons e.g., due to a loss of power, insufficient nebulizing liquid or a failure of the aerosol nebulizer, or may be based on human factors e.g., a physical exhaustion, cough, distractions or other circumstances. Such interruptions may also result in an incorrect adherence evaluation. Further, the aerosol nebulizer may be used in a scenario not associated with nebulizing in accordance with the therapy protocol. Nebulizing data used for the adherence evaluation and having been obtained in such a scenario may be unclassified or unclassifiable nebulizing data, negatively impacting the quality and correctness of the adherence evaluation.

Against this background, it is an objective of the present invention to overcome the above technical disadvantages.

BRIEF EXPLANATION OF THE DRAWINGS

Fig. 1 shows a schematic illustration of an aerosol nebulizer control device and an aerosol nebulizer.

Fig. 2 shows a schematic illustration of a medical evaluation system.

Fig. 3 shows a flow chart of a medical evaluation method for evaluating a validity of a nebulizing session prescribed by a therapy protocol.

Fig. 4 shows a flow chart of a medical evaluation method for evaluating an adherence to the therapy protocol, taking into account the evaluation according to Fig. 3 and performing the evaluating for nebulizing operations and nebulizing sessions in separate steps.

Fig. 5 shows a flow chart of a medical evaluation method for evaluating an adherence to the therapy protocol, taking into account the evaluation according to Fig. 3 and performing the evaluating for nebulizing operations and nebulizing sessions in a combined step.

Fig. 6 shows a table of examples of nebulizing status codes.

DESCRIPTION OF THE EMBODIMENTS

Embodiments of the present invention are described with reference to the Figures. It is noted that the following description should not be construed as limiting the invention. In the following and the above, similar or same reference signs indicate similar or same elements or operations.

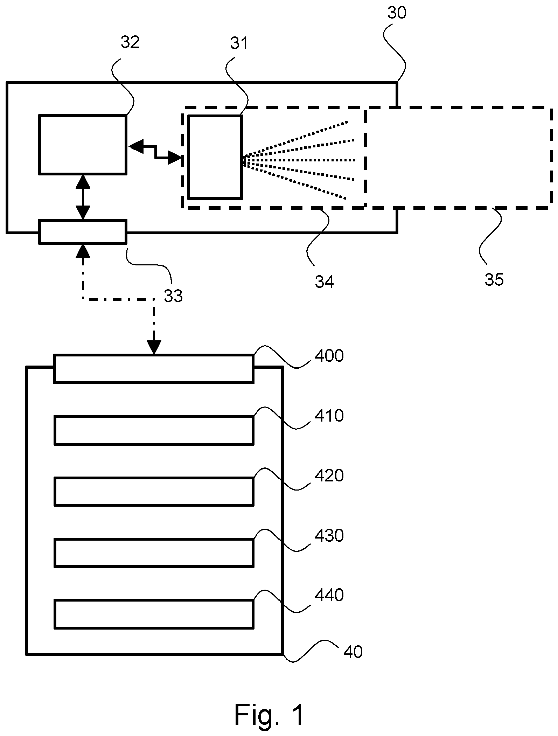

Fig. 1 shows a schematic illustration of an aerosol nebulizer control device for an aerosol nebulizer for implementing the present invention. In particular, an aerosol nebulizer 30 according to Fig. 1 comprises an aerosol generator 31 to generate an aerosol from a liquid medication. Here, the aerosol generator 31 e.g., a membrane aerosol generator or the like, may be a device suitable to nebulize a liquid medication, which is held in a reservoir (not shown) of the aerosol nebulizer 30.

For controlling the operation of the aerosol nebulizer 30, a control device 40 may be used. To improve the usability of the aerosol nebulizers for the user e.g., for the purpose of a smaller size and light-weight handheld portion of the nebulizer, it may be preferrable that the control device 40 is external to the aerosol nebulizer 30 as shown in Fig. 1. The present invention is however not limited thereto and the control device 40 may also be installed internal to or as part of the aerosol nebulizer 30.

Such a control device 40, internal or external, may comprise communication interface 400, a processor 410, a memory 420, for example in the form of a volatile and/or non-volatile memory, a display 430, and/or one or more LEDs and an input unit 440 which may be configured in the form of a keyboard, individual buttons, or a touch-sensitive surface on/in the display. The control device 40 may additionally operate to establish a wired/wireless communication connection with the aerosol nebulizer 30 to transfer configuration data to the aerosol nebulizer 30 to appropriately set and control the operation of the aerosol generator 31. E.g., for improved convenience and mobility, a wireless communication connection may be preferable, but a wired communication connection may also be used for providing the communication connection. When using a wired communication connection, the wire used therefore may also provide further wire-based functions e.g., it may provide power to the aerosol nebulizer 30 if it does not contain its own power source. A wired communication connection may be used to facilitate charging of the aerosol nebulizer 30 whilst providing the communication connection. Nebulizing data indicating an operation of the aerosol nebulizer 30 and/or indicating a usage of the aerosol nebulizer 30 by the user/patient may be generated and transferred via the wired/wireless communication connection from the aerosol nebulizer 30 to the control device 40. Such nebulizing data, which may include nebulizing data and/or measurement data, may be generated, for example, by an operation unit 32 of the aerosol nebulizer 30 and/or one or more sensors that are mounted at the aerosol nebulizer 30 and/or are connected with the aerosol nebulizer 30.

The aerosol nebulizer 30 may be used for nebulizing the liquid medication to allow inhaling of the nebulized liquid medication i.e., an aerosol comprising a drug of the medication. Specifically, the liquid medication may be transformed into an aerosol by the aerosol generator 31, which subsequently enters an inhalation chamber 34 that is connected to a patient interface 35 e.g., a mouthpiece, face mask, nasal prongs or the like. From the patient interface, the patient/user may inhale the aerosol into the respiratory tract to perform a medical treatment of the lungs, throat, nose or sinuses according to an (aerosol) therapy protocol. Further, the aerosol generator 31 may operate according to configuration data that may be provided from a control device 40 via a communication interface 33 and/or an operation unit 32 of the aerosol nebulizer 30. The aerosol nebulizer 30 may provide the nebulizing data, which may indicate the nebulizing operations by including control data and/or measurement data generated by one or more sensors (not shown) at the aerosol nebulizer 30 and/or connected with the aerosol nebulizer 30, via the communication interaction to the control device 40. Examples of such sensors are sensors for detecting a state of a membrane aerosol generator 31, for detecting a filling level of a liquid medication in a reservoir of the aerosol nebulizer 30, for detecting inhalation/exhalation flow/volume/time characteristics during the use of the aerosol nebulizer 30 of the user/patient, and the like.

The control device 40 may establish a communication connection with the medial evaluation device 10 and may perform data (e.g., nebulizing (session) data) communication with the medical evaluation device 10 via the established communication connection. This communication connection may be a wireless connection e.g., via cloud server, a radio connection using a radio communication module, such as a GSM module, UMTS module, LTE module, with or without a SIM card, Wi-Fi module, Bluetooth module, near field module or the like. The present invention is however not limited thereto meaning the communication connection may be a wired connection. The communication connection may be a direct connection to the medical evaluation device 10 or may be facilitated indirectly e.g., by use of computer network equipment such as a modem, router, switch, hub, access point or the like (referred to as "communication device"). Via the established communication connection, a data communication between the control device 40 and the medical evaluation device 10 may be performed so that the control device 40 may send/receive data to/from the medical evaluation device 10. As will be further described below such a data transfer may comprise, for example, the transfer of configuration data for the proper operation of the control device 40 and/or the aerosol generator 31 as well as the transfer of nebulizing data, for example in the form of measurement data and/or inhalation data.

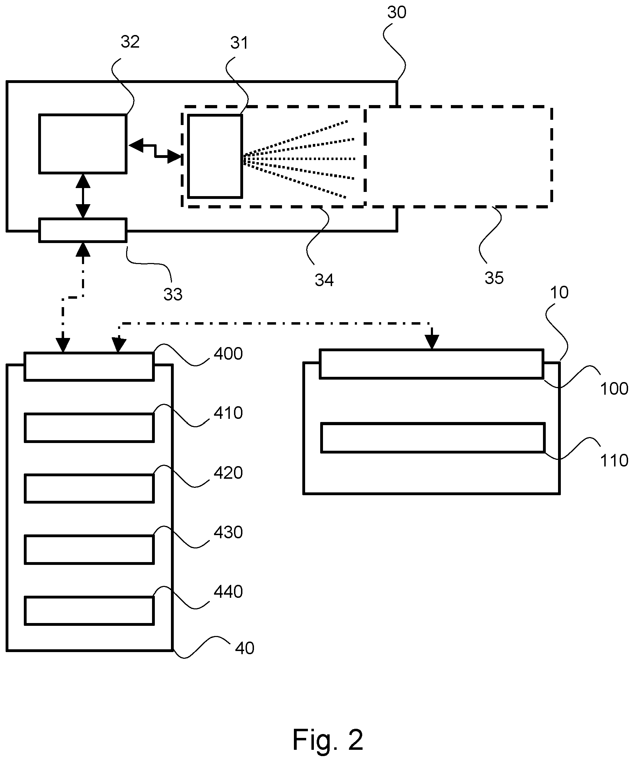

Further, the control device 40 may be a customized communication hub, a portable device such as a smartphone, a tablet, or a smart watch or other smart wearable devices having a customized application program, or the like. The control device 40 may also be a customized communication hub without an input unit and/or a display unit. Further, the control device 40 may receive an instruction from a medical evaluation device 10 e.g., as shown in Fig. 2 (described below), to activate or deactivate the establishment of the communication connection between the nebulizer control device 40 and the medical evaluation device 10. The control device 40 may thus itself be connected e.g., via a cloud server (not shown), with the medical evaluation device 10 or any other centralizing computer to provide feedback for the user/patient so that the inhalation therapy and/or the operation of the aerosol nebulizer 30 are suitably adjusted based on an evaluation performed by an evaluation unit 110 (as described below).

Another example of such an external control device 40 is an external computing device, for example in the form of a smartphone or a PDA as described in DE 102 43 371 A1 or US 2006/0237001 A1. Such an external control device 40 may further exhibit a telecommunication module and may thus further offer the capability to transfer the nebulizing data via the internet to a central data base, for example for telemedicine and medical evaluation purposes and for the purpose of (centralized) electronic health records.

Fig. 2 shows a schematic illustration of a medical evaluation system including an aerosol nebulizer control device and an aerosol nebulizer 30 as shown in Fig. 1 and further including one medical evaluation device 10. Herein, an inhalation process may be evaluated by the remote medical evaluation device 10 with respect to the prescribed therapy protocol e.g., to determine an adherence to the therapy protocol. Herein, the medical evaluation device 10 may comprise a communication unit 100 and an evaluation unit 110. By use of such a medical evaluation device 10, users/patients may obtain medical feedback informing whether the inhalation therapy has been performed compliant to the therapy protocol. Such an approach may improve adherence to the therapy protocol by an improved evaluation process using telemedicine capabilities, for example taking into account confidentiality and integrity of personal health data as well as reliable technical feedbacks.

The evaluation unit 110 of the medical evaluation device 10 may also be referred to as a (second) processor, and the medical evaluation device 10 may be e.g., a centralized server or a distributed computer network.

The communication unit 100 of the medical evaluation device 10 may be configured to establish a communication connection with the control device 40 (as described above). The communication connection may be configured for providing first configuration data to the aerosol nebulizer 30 e.g., generated by the evaluation unit 110, via the nebulizer control device 40 (and optionally the above-mentioned communication device). Here, the first configuration data may be for configuring the aerosol nebulizer 30 according to the prescribed (aerosol) therapy protocol. E.g., the first configuration data may include at least one of a current date, a current time, a predetermined (maximal/minimal) nebulizing duration, a number of maximal storable records, possible modes of the device to be selected by the patient, an activation or a deactivation of the automatic data storage and transfer, an identification of one or more drugs usable for said liquid medication or the like. The first configuration data may also include an identification value of the control device 40, because each data transfer preferably is performed including a check whether the identification values match with a pre-defined association (e.g., a whitelist) e.g., as described in WO 2017/137424 A1.

The communication connection in Fig. 2 between the control device 40 and the communication unit 100 of the medical evaluation device 10 may be further provided to receive nebulizing data indicating a nebulizing operation of the aerosol nebulizer 30. The nebulizing data may further include inhalation data that indicate a usage of the aerosol nebulizer 30 by the user/patient. For example, the nebulizing data may include at least one of a nebulizing date, a nebulizing time, a nebulizing duration, one or more interruption criteria for switch-off of the aerosol nebulizer, usage status by a user/patient, a fill level of the liquid medication in the reservoir, inhalation/exhalation flow/volume/time characteristics of said user, and a list of one or more drugs used or to be used for said liquid medication. Such nebulizing data may be appropriately generated by the operation unit 32 of the aerosol nebulizer 30 e.g., by an appropriate processing of acquired sensor signals.

Further, the evaluation unit 110 of the medical evaluation device 10 may be configured to perform an evaluation of the nebulizations performed by the user/patient by use of nebulizing data provided by the aerosol nebulizer 30. This evaluation may be based on a comparison of said received nebulizing data with respective data/parameters that are defined within the prescribed therapy protocol. E.g., as will be described in further detail below, the therapy protocol may define a minimum inhalation duration parameter which indicates a time duration which is considered to be required to effectively perform an inhalation with a particular medical drug. Such parameter may (also) be the number of inhalations that should be performed on a single day. Accordingly e.g., the evaluation unit 110 may perform a processing in which the received nebulizing data are assigned to respective (stored) parameters defined for a prescribed (user-specific) therapy protocol, and subsequently a comparison is performed to evaluate whether the received nebulizing data matches with the required parameter/s. If such a matching occurs e.g., if the user/patient has performed an inhalation process that is sufficiently long as defined by the minimum inhalation duration parameter, then this inhalation process may be determined to be a valid inhalation process. Otherwise e.g., if the user/patient has performed an inhalation process that is not sufficiently long as defined by the minimum inhalation duration parameter, then this inhalation process may be determined to be an invalid inhalation process.

In this context, the terms "valid" and "invalid" may indicate whether an inhalation process, or a nebulizing operation/session (as described below), may be used for an evaluation processing following the obtaining of data. Such processing may relate to e.g., deciding whether clustering of nebulizing operations should be performed and/or evaluating an adherence to the therapy protocol based on the (clustered) nebulizing operations. More details of such processing are described below.

As a result of the above when performing the evaluation, the evaluation unit 110 may further generate feedback data, such as therapy related feedback data. The generated therapy related feedback data may subsequently be transmitted by the communication unit 100 via the established communication connection to the control device 40.

The therapy related feedback data may visually and/or audibly indicate at the nebulizer control device 40 that the user/patient has not followed the therapy protocol as prescribed (e.g., based on a certain set of parameters that are set for the user) and urges or reminds the user/patient to appropriately change the usage of the aerosol nebulizer 30.

As shown in Fig. 2, the control device 40 may be provided separately from the aerosol nebulizer 30 in order to separate the control functionality as much as possible from aerosol nebulizer 30. This is, however, not considered to be limiting and the control device 40 may alternatively be provided as an integral part of the aerosol nebulizer 30, for example in the form of an integral unit of the aerosol nebulizer, for example as a suitably adapted operation unit 32. In such a case, the communication interface 33 would be configured to establish a wireless communication interaction (as explained below) with the medical evaluation device 10.

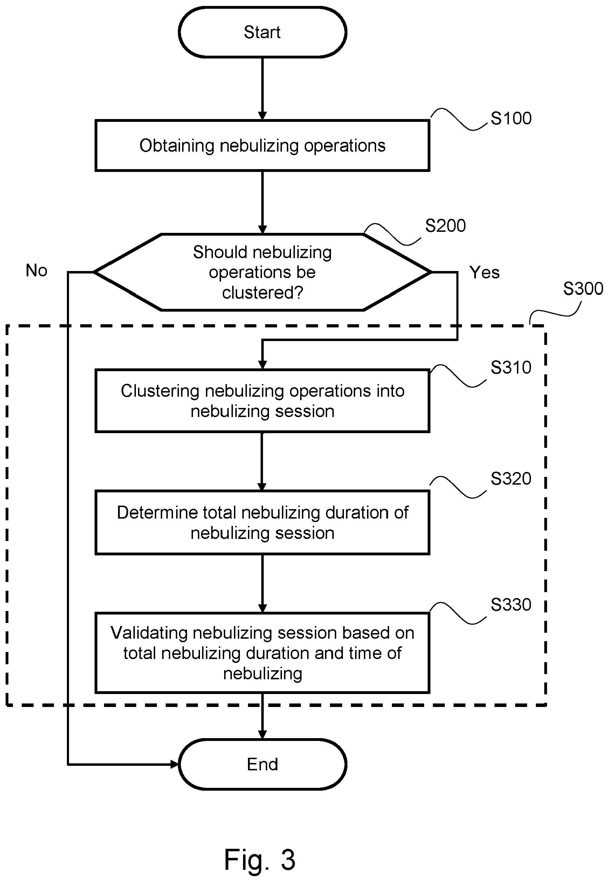

Fig. 3 shows a flow chart of a medical evaluation method for evaluating a validity of a nebulizing session prescribed by the therapy protocol. Here, the therapy protocol may be a series of instructions indicating how and/or when a medication or combination of medications should be consumed by the user/patient through use of the aerosol nebulizer 30. The therapy protocol may define these instructions as one or more nebulizing durations e.g., as seconds, minutes or hours, for one or more nebulizing times/moments e.g., as a specific/general time of a day. Here the nebulizing duration may be based on the dosage provided to the aerosol nebulizer 30 and the speed at which this dosage may be nebulized into aerosol.

The medical evaluation method of Fig. 3 may be performed by the operation unit 32 of the aerosol nebulizer 30, the processor 410 of the control device 40 or the medical evaluation device 10 as shown in Figs. 1 or 2, or any other processor of a computing device, or a combination thereof. Here, the medical evaluation method for evaluating the validity of a nebulizing session prescribed by a therapy protocol when using an aerosol nebulizer 30 comprising an aerosol generator 31 for nebulizing a liquid medication may comprise the following steps S100, S200 and S300.

Step S100: obtaining nebulizing data indicating (at least) a first nebulizing operation and a second nebulizing operation of the aerosol nebulizer. Herein, each of the first and second nebulizing operation may have an individual nebulizing duration at an individual nebulizing time and a nebulizing status code e.g., for a predetermined liquid medication.

S200: deciding whether to perform clustering, based on the individual nebulizing time and/or the nebulizing status code (including potentially also the predetermined liquid medication or nebulized drug) and/or the individual nebulizing duration of the first and/or the second nebulizing operation and/or the liquid medication or drug to be nebulized with the first and/or second nebulizing operation.

Step S300: conditional clustering or grouping (the terms may be used interchangeably) of the nebulizing operations into the nebulizing session and validating of the nebulizing operation and/or nebulizing session or cluster (the last two terms may be used interchangeably). If it is decided (Step S200: Yes) to perform clustering, Step S300 is entered and comprises clustering (Step S310) of the first and second nebulizing operation into the (common) nebulizing session, determining (Step S320) of a total nebulizing duration for the nebulizing session based on the individual nebulizing duration of the first and second nebulizing operation clustered into the nebulizing session, and evaluating (Step S330) of the validity of the nebulizing session based on a prescribed nebulizing duration specific to the therapy protocol and the total nebulizing duration determined for the nebulizing session, and the prescribed/total nebulizing duration preferably is of a duration of more than or equal to four minutes or more than or equal to one minute.

For conciseness, the present disclosure exemplifies the present invention by reference to two nebulizing operations i.e., the first and second nebulizing operation mentioned above. However, the present invention is not limited thereto and two or more, e.g., three or more, or four or more nebulizing operations may be clustered. The Steps S100 to S300 (and Steps S400 to S700 that will be described below), mutually apply to such two or more nebulizing operations e.g., a third and/or fourth nebulizing operation. That is to say, the nebulizing data may indicate a plurality of nebulizing operations (e.g., including the first and second nebulizing operation mentioned above and, optionally, a third nebulizing operation and/or a fourth nebulizing operation) of the aerosol nebulizer, each of the plurality of nebulizing operations having an individual nebulizing duration at an individual nebulizing time and a nebulizing status code. In this case, the method may comprise deciding (i.e., Step S200) whether to perform clustering, based on the individual nebulizing time and/or the nebulizing status code and/or the individual nebulizing duration of at least one of the plurality nebulizing operations and/or the liquid medication to be nebulized in the at least one of the plurality of nebulizing operations. If it is decided to perform clustering, the method may further comprise clustering (i.e., Step S310) at least two of the plurality of nebulizing operations into the nebulizing session, determining (i.e., Step S320) a total nebulizing duration for the nebulizing session based on the individual nebulizing duration of the at least two of the plurality of nebulizing operations clustered into the nebulizing session, and evaluating (i.e., Step S330) the validity of the nebulizing session based on a prescribed nebulizing duration specific to the therapy protocol and the total nebulizing duration determined for the nebulizing session.

As will be described below in more detail (e.g., in reference to Steps S500 and S600), without clustering i.e., after deciding (Step S200: No) not to perform clustering, the single nebulization operation may be evaluated regarding its validity based on the above mentioned criteria.

After steps S100, S200 and S300 the evaluation method may end.

In Step S100, the nebulizing data may be obtained by the operation unit 32 of the aerosol nebulizer 30 (e.g., in conjunction with one or more sensors (not shown)), the processor 410 of the control device 40 or the medical evaluation device 10 (e.g., by transmitting such nebulizing data via the communication interface 33 or the communication interface 400). An individual nebulizing duration may be a period of time indicating how long the nebulizing function of the aerosol nebulizer 30 e.g., the aerosol generator 31, is activated. E.g., the individual nebulizing duration may be a time period (e.g., in milliseconds, seconds or minutes) indicating how long the individual nebulizing operation lasts or how long the user/patient is busy performing/doing the inhalation. An individual nebulizing time may be an indication of a moment of time when the nebulizing function of the aerosol nebulizer 30 e.g., the aerosol generator 31, is activated or deactivated to inform when a corresponding individual nebulizing duration took place. E.g., the individual nebulizing time may be a timestamp indicating when the individual nebulizing operation took place (e.g., being a time at which nebulizing begins or ends). The nebulizing status code may indicate whether:

- the nebulizing operation has finished as expected;
- the aerosol nebulizer 30 has lost power;
- the nebulizing operation has been started without providing the liquid medication;
- the aerosol nebulizer 30 has been manually shutdown;
- the aerosol nebulizer 30 has stopped due to a failure mode; and/or
- a timeout has been reached,

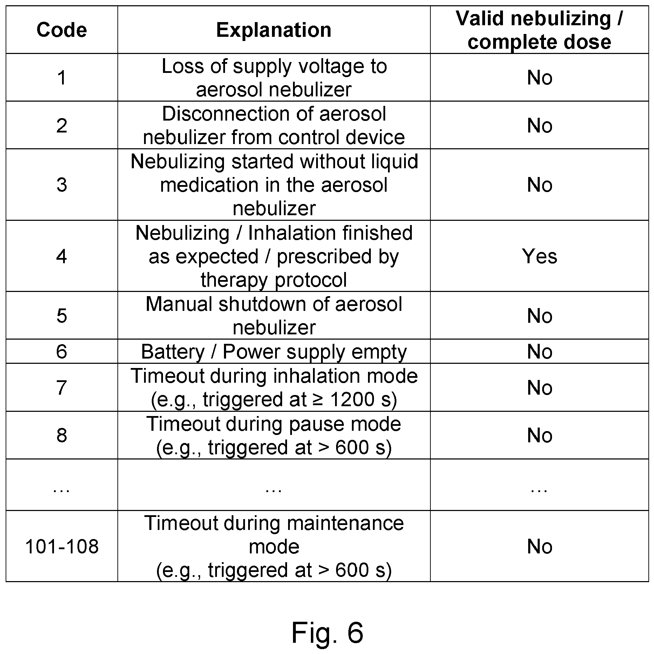

and may be stored as part of metadata associated with the nebulizing data and/or data encoding the individual nebulizing duration and individual nebulizing time of the nebulizing operation. Here, the status code may be a value as exemplified in Fig. 6.

As such, a nebulizing operation may indicate an operation of the aerosol generator 31 for an individual nebulizing duration e.g., between a start time of the aerosol generator 31 operation and a switch-off or end time of the aerosol generator 31 operation. Here, the start time and end time or switch-off time are examples of the individual nebulizing time. The nebulizing status code(s) may identify individual causes or criteria for aerosol generator/nebulizer switch-off or shut-down and are further detailed below. A nebulization duration may also be a sum of duration of single nebulization operations while the patient is inhaling continuously through the device. A nebulization may or may not include a pause taken by the patient during the nebulization session.

Further, the nebulizing data may include inhalation data and/or measurement data generated by one or more sensors (not shown) at the aerosol nebulizer 30 and/or connected with the aerosol nebulizer 30. Examples of such sensors are sensors for detecting a state of a membrane aerosol generator, for detecting a filling level of a liquid medication in a reservoir of the aerosol nebulizer, for detecting inhalation/exhalation flow/volume/time characteristics during the use of the aerosol nebulizer of the user/patient, contact of the patient with the nebulizer device and the like. The nebulizing data may further include a usage status by a user, a fill level of the liquid medication in the reservoir, inhalation/exhalation flow/volume/time characteristics of said user, and a list of one or more drugs or medication used or to be used for said liquid medication. Such nebulizing data may be appropriately generated by the operation unit 32 of the aerosol nebulizer 30, for example by an appropriate processing of acquired sensor signals.

Further in this context, nebulizing data may (also) include respective nebulizing/inhalation start date/times, nebulizing/inhalation end date/times, and nebulizing durations for successive sessions/operations of said aerosol nebulizer 30. Based on the received nebulizing data, it may be determined whether a minimum inhalation duration, as prescribed by the therapy protocol, is reached by the nebulizing session/s or nebulizing operation/s of said aerosol nebulizer 30 e.g., to inform about whether a nebulizing/inhalation session/operation of the liquid medication is actually performed for the required minimum inhalation duration.

Further in this context, a nebulizing operation may be associated with an inhalation process and/or with operating the aerosol generator of the aerosol nebulizer e.g., to produce aerosol from the liquid medication. After all, it may not always be possible to directly obtain information about the inhalation behavior/performance of the user/patient. Therefore, information e.g., obtained from the nebulizing operation/s such as the individual nebulizing duration, the individual nebulizing time and the nebulizing status code may be used to infer how the user/patient inhales.

In Step S200, the deciding may be involve determining whether a predetermined condition, a predetermined threshold and/or a predetermined e.g., regarding the individual nebulizing time, the individual nebulizing duration and/or the nebulizing status code (as described above) is/are met. Thereby, it may be decided whether the nebulizing operations obtained from the nebulizing data need clustering for subsequent evaluation processing or not. This means, the clustering is conditional based on the deciding whether to perform clustering. E.g., if different liquid medications / drugs are nebulized by the first and second nebulizing operation mentioned above, it may be decided (Step S200: No) not to perform clustering. Further examples for deciding whether to perform clustering or whether to include a nebulizing operation into the nebulizing session (the two phrases may be used interchangeably) is described below.

In Step S310, the clustering may also be referred to as grouping, collecting or combining of nebulizing operations. Thereby, nebulizing operations may be treated together or seen as being dependent or related to each other instead of treating them individually or independent from each other. This may allow a subsequent evaluation processing to be based on valid or complete data represented by the nebulizing session, instead of individually evaluating invalid or incomplete nebulizing operations independently from each other.

E.g., the deciding whether to perform clustering may involve deciding whether to include a nebulizing operation into the nebulizing session based on e.g.:

a) whether nebulizing of a dose of the liquid medication has completed as expected or not; or
b) whether a supply voltage was lost; or

c) whether the aerosol nebulizer (or a component thereof) was disconnected when nebulizing; or

d) whether nebulizing was started without liquid medication in the reservoir of the aerosol nebulizer 30; or

e) whether the aerosol nebulizer 30 was manually shut down; or

f) whether the battery or power source of the aerosol nebulizer 30 was empty; or

g) whether the aerosol nebulizer timed out during nebulizing mode e.g., after 10 to 30 minutes e.g., 1200 seconds; or

h) whether the aerosol nebulizer 30 timed out during a pause mode e.g., after 600 seconds; or

i) whether the aerosol nebulizer 30 timed out during a maintenance mode (which may also be referred to as backflushing mode, regeneration mode, device care mode or cleaning mode) e.g., after 600 seconds; or

j) whether the nebulized liquid medication was the same or was different in the two nebulization operations; or

k) whether the aerosol nebulizer was switched off / powered off due to a failure mode.

The above list of cases a) to g) may be indicated by the nebulizing status codes as shown in Fig. 6. The number of cases is however not limited thereto and more or fewer cases (and nebulizing status codes) may be provided. For the above cases, the nebulizing operation corresponding to cases a), d), h), i) and j) may not be clustered into i.e., excluded from the nebulizing session and subsequent evaluation processing, and cases b), c), e), f) and g) may be clustered into i.e., included in the nebulizing session and subsequent evaluation processing. Specifically, for case j) if the liquid medication for the first nebulizing operation is different to the liquid medication for the second nebulizing operation, it may be decided not to perform clustering.

In Step S320, the total nebulizing duration for a nebulizing session may be a (weighted) sum of the individual nebulizing durations of each nebulizing operation clustered into the nebulizing session. If the individual nebulizing durations are not provided as part of the nebulizing data, the total nebulizing duration may be obtained by taking into account the individual nebulizing time (e.g., start time and end time) of each nebulizing operation clustered into the nebulizing session, and at least one period of inactivity or non-performing of nebulizing between successive nebulizing operations may be subtracted from the duration from the start of the first until the end of the last nebulizing operation clustered into the nebulizing session. It is however preferred to include the individual nebulizing duration in the nebulizing data to reduce the amount of processing necessary to obtain the total nebulizing duration.

In Step S330, the validity of nebulizing (e.g., of a nebulizing session or nebulizing operation) is evaluated. This validity may be indicative of whether to treat the nebulizing session (or nebulizing operation/s) as "valid" e.g., for use in subsequent evaluation processing (e.g., adherence evaluating as will be described below). Specifically, the validity may relate to whether a prescribed medication dosage is completely consumed by the user/patient (e.g., as prescribed by the therapy protocol). It may also relate to whether a prescribed medication dosage is consumed during a prescribed period (e.g., during a prescribed nebulizing duration). It may also relate to whether the drug dosage is in/sufficient, in/complete or negligible (e.g., indicative by an operation of the aerosol generator sufficiently long to generate aerosol). It may also relate to whether nebulizing has been performed after a predetermined break, wait or pause period (e.g., the prescribed duration between nebulizing is waited before starting a subsequent nebulizing). It may also relate to whether nebulizing has been performed within a corresponding therapy window (e.g., according to the therapy protocol and allowing a single nebulizing operation/session to take place within the duration of a therapy window only). The validity or aspects related thereto may (also) be indicated by the nebulizing status code, as shown in Fig. 6.

E.g., the therapy protocol may prescribe to perform nebulizing at least twice throughout the day with a sufficient break, wait or pause period therebetween e.g., to maintain a minimum concentration of the medication. An optimal break, wait or pause period may be twelve hours, but may be as short as e.g., six or eight hours. Therefore, if nebulizing is performed without waiting for the prescribed break, wait or pause period the nebulizing operation/session may be evaluated as invalid. In this example, the individual nebulizing time of the nebulizing operations or the nebulizing time of nebulizing sessions may indicate that nebulizing takes place without conforming to the break, wait or pause period. Such a nebulizing operation/session may not be used for subsequent evaluation processing (e.g., the adherence evaluating which will be described below) as doing so may result in an incorrect conclusion that the nebulizing took place in accordance with the therapy protocol, although this was not actually the case.

E.g., the therapy window may be a time window lasting six or eight hours. E.g., if two (un-clustered) nebulizing operations or two nebulizing sessions are identified as taking place during the same therapy window, it may be determined that at least one of the two nebulizing operations or of the two nebulizing sessions is not valid or invalid.

It is worth mentioning that the break, wait or pause period or the therapy window may be used to determine whether the time/temporal spacing between a nebulizing operation and a nebulizing session (i.e., clustered nebulizing operations) is sufficient. That is to say, Step S330 is not limited to comparing the time/temporal spacing to nebulizing sessions and Step S500 is not limited to comparing the time/temporal spacing to nebulizing operations. Instead, the time/temporal spacing to any (previous) nebulizing operation/session may be considered when evaluating whether a nebulizing operation/session is valid.

This means, the evaluating (Step S330 and Step S500; the latter will be explained in more detail below) the validity may be based on a time/timely/temporal distance/spacing between two nebulizing sessions, two nebulizing operations or a nebulizing session and a nebulizing operation or vice versa. E.g., if the timely distance is below a predetermined (minimum) threshold, it may be determined that the nebulizing operation and/or nebulizing session is not valid or invalid.

Based on the above, the medical evaluation method can take into account that, if a plurality of nebulizing operations are obtained (e.g., due to an issue with the communication connection, due to an event having occurred at the aerosol nebulizer 30, due to a physical or circumstantial impact, or due to any other reason as outlined above), there may be situations where the plurality of nebulizing operations should not be treated individually as doing so may result in an incorrect association with the nebulizing according to the therapy protocol. Specifically, if e.g., the individual nebulizing operations are too short individually but took place due to interruption of the inhalation process (e.g., due to technical reasons, because the user/patient started coughing, or the like), took place at a timing not specified by the therapy protocol, or were produced when the aerosol nebulizer 30 is not used for nebulizing according to the therapy protocol, any subsequent analysis or evaluation based on such nebulizing operations would be of low quality and/or incorrect.

Therefore, (conditionally) clustering the nebulizing operations into a common nebulizing session may allow treating the individual (but clustered) nebulizing operations as one continuous "virtual nebulizing operation", corresponding to nebulizing in accordance with the therapy protocol. Subsequent evaluating of whether the nebulizing session (or the virtual nebulizing operation) is valid in view of the therapy protocol makes possible the providing of valid nebulizing data to subsequent evaluation processing (e.g., an adherence evaluation as described below).

In other words, clustering or grouping the nebulizing operations into a common nebulizing session can be understood as a conditional combination of nebulizing data of individual nebulizing operations into one data unit indicating a total nebulizing duration corresponding to the individual nebulizing operations despite the fact that there is a time gap between the individual nebulizing operations.

For example, a patient or user may start a first nebulizing operation, the nebulizing gets interrupted because of loss of power, coughing or the like (see examples of error codes in Fig. 6), and then the user starts a second nebulizing operation, then the first and second nebulizing operations (and the corresponding first and second nebulizing data) may conditionally be clustered or grouped into a single common nebulizing session.

The condition used to decide whether (or not) to perform the clustering may be based on the considerations such as when the individual nebulizing operations take place, how long it lasts, which medication was used, the time between the individual nebulizing operations, and/or which nebulizing status code is associated thereto. More specifically, the condition may be based on the following considerations.

According to a preferred embodiment, the first and second nebulizing operation may be clustered into the common nebulizing session if the individual nebulizing time of each of the first and second nebulizing operation lies within a predetermined allowable nebulizing session time window. Here, the allowable nebulizing time window may be specific to the liquid medication, and preferably may be of a duration of up to two hours. The allowable nebulizing time window may (also) be based on a predetermined minimum in vivo concentration, e.g., a (blood) concentration of a drug of the liquid medication in the user/patient after inhaling according to the therapy protocol. This concentration of the liquid medication may be obtainable when distributing a medication dosage of one nebulizing operation lasting the prescribed nebulizing duration over a medication intake period predetermined by the therapy protocol. E.g., a scenario is assumed, wherein the therapy protocol prescribes a valid nebulizing operation (or valid nebulizing session) to take place during an allowable nebulizing time window of up to two hours starting from 9:00 o'clock in the morning, wherein the prescribed medication must be inhaled for at least ten minutes. In this scenario, if the individual nebulizing time of each of the first and second nebulizing operation falls within this allowable nebulizing time window of two hours, then clustering into the nebulizing session may be performed.

According to another preferred embodiment, the first and second nebulizing operation may be clustered into the nebulizing session if an inactivity period between the first and second nebulizing operation is less than or equal to a predetermined inactivity period. Here, the predetermined inactivity period may be specific to the liquid medication, and may preferably be of a duration of less than or equal to thirty minutes, less than or equal to fifteen minutes, less than or equal to ten minutes, or less than or equal to five minutes. Such an inactivity period may be defined as a time interval from the end of the first nebulizing session to the start of the subsequent second nebulizing session. Thereby, certain breaks or interruptions of the nebulizing may be omitted or discarded. This may be the case for breaks or interruptions that do not have any or have negligible impact on the absorption and/or effect of the drug of the liquid medication. In line with the scenario assumed above, it may be assumed e.g., that the first nebulizing operation is started according to the therapy protocol after 9:00 o'clock but the aerosol nebulizer 30 runs out of power before the individual nebulizing duration reaches the prescribed minimum nebulizing duration of ten minutes. When the first nebulizing operation stops, the predetermined inactivity period begins. If the nebulizing is resumed within this predetermined inactivity period e.g., by starting the second nebulizing operation up to thirty minutes after the first nebulizing period ended, clustering into the nebulizing session may be performed.

According to another preferred embodiment, the therapy protocol may specify at least two nebulizing sessions as having to take place within a predetermined therapy period assigned to each of the at least two nebulizing sessions. E.g., in addition to a first nebulizing session with a (first) allowable nebulizing time window of up to two hours starting at 9:00 o'clock in the morning, wherein the prescribed medication must be inhaled for at least ten minutes (i.e., like the scenario assumed above), the therapy protocol may further specify or prescribe (the terms may be used interchangeably) a second nebulizing session with a (second) allowable nebulizing time window of up to one hour starting from 13:00 o'clock in the afternoon, wherein the prescribed medication must be inhaled for at least ten minutes. A (first) therapy period of the first nebulizing session may be from 9:00 until 13:00 o'clock, wherein the allowable nebulizing time window may be flexibly located within this therapy period, and a (second) therapy period of the second nebulizing session may be from 13:00 o'clock until the evening, night or end of day, wherein the allowable nebulizing time window may be flexibly located within this therapy period. Thereby, a plurality of nebulizing operations recorded over the predetermined data recording period mentioned above, may be selectively clustered into one of the at least two nebulizing sessions e.g., taking into account the therapy period of the at least two nebulizing sessions.

According to another preferred embodiment, the first and second nebulizing operation may be clustered into the first nebulizing session of the at least two nebulizing sessions if the individual nebulizing time of each of the first and second nebulizing operation is before the therapy period of the second nebulizing session of the at least two nebulizing sessions. E.g., the first and second nebulizing operation may be clustered into the second nebulizing session of the two or more nebulizing sessions if the individual nebulizing time of each of the first and second nebulizing operation is after the (first) therapy period of the first nebulizing session and after or during the (second) therapy period of the second nebulizing session. Here, the first nebulizing session may be before the second nebulizing session. Thereby, one or more nebulizing operations of a plurality of nebulizing operations may be correctly clustered into one nebulizing session (preferably taking into account the allowable nebulizing time window and the predetermined inactivity period). Subsequent validity evaluation (and/or adherence evaluation) may therefore be performed on valid nebulizing sessions (or valid nebulizing operation/s e.g., if the corresponding nebulizing operation/s is/are not clustered into a nebulizing session).

Further to the condition for (positively) deciding that clustering should be performed, an (additional) condition may be used that a nebulizing operation (e.g., such as the first and second nebulizing operation above) may not or should not be clustered into a nebulizing session. More specifically, such a condition may be based on the following considerations.

According to a preferred embodiment, the deciding of whether or not to cluster (Step S200) may comprise not to perform clustering into the nebulizing session if the nebulizing duration of the first and/or second nebulizing operation is shorter than a predetermined minimum nebulizing duration. Here, the minimum nebulizing duration may be a duration corresponding to an accidental activation of the aerosol nebulizer 30 or a duration so short that it is not associable with any nebulizing according to the therapy protocol. E.g., the minimum nebulizing duration may be one minute or less, fifteen seconds or less, or ten seconds or less. Thereby, nebulizing operations not associable with nebulizing (e.g., because they are too short to produce any meaningful amount of aerosol) according to the therapy protocol may not be clustered and (preferably) omitted or filtered out from subsequent evaluation processing (e.g., validity and/or adherence evaluation) altogether.

The evaluating the validity of a (single) nebulizing operation and/or of a (clustered) nebulizing session (Step S500 and/or S330) may (also) comprise evaluating if the individual nebulizing duration and/or the total nebulizing duration (e.g., of the nebulizing operation and of the nebulizing session, respectively) is longer than the predetermined minimum nebulizing duration. E.g., there may be a case wherein the individual nebulizing duration of a nebulizing operation is too short for the nebulizing operation to be clustered into a nebulizing session (e.g., Step 210: No). In this case, the individual nebulizing duration may also be too short for the nebulizing operation to be taken into account for subsequent evaluation processing (e.g., the adherence evaluation in Step S600). Conversely, there may be a case wherein the individual nebulizing durations of nebulizing operations are long enough to be clustered into a nebulizing session (e.g., Step S200: Yes). In this case, the total nebulizing duration may however (still) be too short for the nebulizing session to be taken into account for subsequent evaluation processing (e.g., the adherence evaluation in Step S400). E.g., two nebulizing operations may last 20 seconds each and may be longer than a (first) predetermined minimum nebulizing duration specific to nebulizing operations. As a result, they may be clustered into a nebulizing session, but the total nebulizing duration of 40 seconds may (still) be shorter than a (second) predetermined minimum nebulizing duration specific to nebulizing sessions. That is to say, the predetermined minimum nebulizing duration may be different for nebulizing operations and nebulizing session.

By evaluating the validity with respect to the predetermined minimum nebulizing duration explained above, a nebulizing operation and/or nebulizing session may be determined as valid (e.g., if longer) to include it in subsequent evaluation processing, or not valid or invalid (e.g., if shorter) to exclude it from the subsequent evaluation processing.

According to another preferred embodiment, the deciding according to Step S200 may comprise deciding not to perform clustering into the nebulizing session if the nebulizing status code of the first and/or second nebulizing operation indicates that a nebulizing operation has finished as expected or was done with different medications, if this additional information is available. In a scenario, where one nebulizing operation is completed according to the therapy protocol (e.g., has a duration of ten minutes and took place during the first therapy period described above) the nebulizing status code may indicate that the nebulizing operation finished or has been completed as expected and needs not be clustered with other nebulizing operations. Therefore, already valid nebulizing operations are not clustered with other nebulizing operations that may be invalid or may have to be clustered with yet other nebulizing operations into a separate nebulizing session.

According to another preferred embodiment, the deciding according to Step S200 may comprise not to perform clustering into the nebulizing session if nebulizing status code of the first and/or second nebulizing operation indicates that a timeout has been reached, preferably when the aerosol nebulizer is in an inhalation mode, a pause mode and/or a maintenance mode. Thereby, if a nebulizing operation took place in a scenario not associated to nebulizing according to the therapy protocol, this nebulizing operation may be omitted from further analysis or evaluation processing.

Further, user/patient feedback may be used to control the clustering e.g., allowing the user/patient to (temporarily) disable or deactivate the clustering to always perform an evaluation processing based on the nebulizing operations as such and not on a nebulizing session into which a plurality of nebulizing operations is clustered. According to a preferred embodiment, the evaluation method may further comprise providing an enquiry notification of whether the first and/or second nebulizing operation is to be clustered into the nebulizing session with the second and/or first nebulizing operation, such that the first and second nebulizing operation are clustered into the nebulizing session (only) if the enquiry notification is confirmed (or such that the first and second nebulizing operations are not clustered into the nebulizing session if the enquiry notification is unconfirmed). Here, the enquiry notification may be an audible, a haptic or a visual notification provided by use of the control device 40 or by use of a corresponding notification unit part of the aerosol nebulizer 30. A loudspeaker, a vibrating element, a display, a LED or any other form of notification may be used by the control device 40 or the aerosol nebulizer 30. The feedback or confirmation provided by the user/patient may be obtained by use of a button or gesture (e.g., by moving the control device 40 or the aerosol nebulizer 30 in a predetermined way).

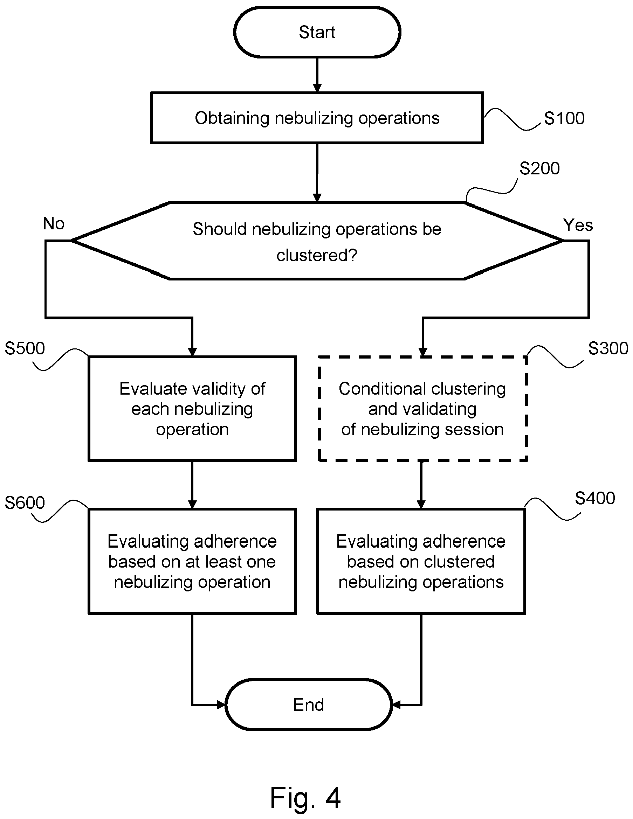

As mentioned above, the medical evaluation method as shown in Fig 3 may end after deciding not to perform clustering or after having evaluated the validity of the nebulizing session. However, as shown in Fig. 4, if it is decided not to perform clustering in Step S200, the medical evaluation method may further comprise evaluating (Step S500) a validity of the (first and/or the second) nebulizing operations based on: a) the prescribed nebulizing duration specific to the therapy protocol and the individual nebulizing duration of the first and/or second nebulizing operation; or b) the prescribed nebulizing duration specific to a shortest possible nebulizing duration of more than one possible nebulizing durations, each prescribed by the therapy protocol for each of more than one liquid medications, and the individual nebulizing duration of the first and/or second nebulizing operation. Herein, the therapy protocol may be based on a specific liquid medication. Therefore, the validity evaluating of nebulizing operations may take into account a sensible prescribed (minimum/medication-specific) nebulizing duration, regardless of whether the liquid medication is identified for the nebulizing operation or not.

E.g., for case a), if the therapy protocol specifies that the user should perform nebulizing (for a specific liquid medication) for a period of ten minutes, a nebulizing operation with an individual nebulizing duration shorter than this so called "prescribed nebulizing duration" may be evaluated as invalid, and a nebulizing session with an individual nebulizing duration longer than or equal to the prescribed nebulizing duration may be evaluated as valid. In this case, the prescribed nebulizing duration may be related to the liquid medication.

The user may use the aerosol nebulizer 30 to inhale one of a plurality of liquid medications, different from each other. For each of said different liquid medications a different nebulizing duration may be prescribed by the therapy protocol. Therefore, the user may interact with the aerosol nebulizer 30 and/or the control device 40 to select which of the liquid medications is/will be nebulized. Based thereon, a corresponding therapy protocol may be used to obtain a medication-specific (minimum) nebulizing duration as the above-mentioned prescribed nebulizing duration.

There may however be a case, where one of a plurality of drugs/liquid is to be inhaled, but the correct drug-specific (minimum) nebulizing duration cannot be obtained. A reason for this may be, that the user forgot to specify the liquid medication, or that the aerosol nebulizer 30 and/or the control device 40 cannot obtain the medication-specific (minimum) nebulizing duration for the selected liquid medication. As a result e.g., according to case b), out of possible (minimum/medication-specific) nebulizing durations each corresponding to one of the plurality of liquid medications, the shortest possible (minimum/medication-specific) nebulizing duration may be chosen as the prescribed nebulizing duration. E.g., if liquid medications A, B and C with a medication-specific nebulizing duration of respectively twelve minutes, ten minutes and fifteen minutes were specified, the prescribed nebulizing duration may be ten minutes in a case where it is not specified which of the liquid medications A, B or C is nebulized. Therefore, even if the liquid medication (to be) nebulized is not/cannot be selected a sensible (minimum/medication-specific) nebulizing duration for evaluating the validity of a nebulizing operation can be chosen/selected/set.

Therefore, if it is decided that the individual nebulizing operations should not be clustered (e.g., because they are long enough individually, if the nebulizing starts without medication in the chamber, if the time interval between the first and second nebulizing operation (i.e. time interval between the end time of the first nebulizing operation and the start time of the second nebulizing operation) is longer than a predetermined time interval), they may individually be used for subsequent evaluation processing.

After all, a plurality of valid or complete nebulizing operations may be obtained e.g., at the end of a predetermined data recording period (e.g., one or more days, weeks or months), making it unnecessary to cluster the (already) valid or complete nebulizing operations. In this case, the nebulizing data may be obtained at/after the end of the data recording period and may indicate the plurality of nebulizing operations as being distributed throughout the data recording period. Such a scenario may be beneficial if the analysis or evaluation should be delayed until sufficient data is obtained. Thereby, invalid nebulizing sessions and/or invalid nebulizing operations may be filtered out and transmitted at once e.g., as a burst transmission, resulting in fewer data transmissions and less data to be transmitted. Further, when transmitting nebulizing (session) data indicating valid nebulizing sessions and/or valid nebulizing operations to a (remote) medical evaluation device 10, this data may be packeted, encrypted and/or compressed data to reduce the number of transmissions and improve privacy.

That is to say, in the above scenario, clustering all the individual nebulizing operations into a nebulizing session may be erroneous and may result in a low quality or incorrect evaluation processing. Therefore, it may be preferrable to perform the clustering on a condition e.g., based on the individual nebulizing time, the nebulizing status code and/or the individual nebulizing duration of the first and/or the second nebulizing operation.

To tie in the above exemplifying conditions with the validity evaluation described further above (i.e., Step S330 and S500), if clustering is performed and each of the first and second nebulizing operation has an individual nebulizing duration of e.g., five minutes, the total nebulizing duration (of the nebulizing session or virtual nebulizing operation) would be ten minutes. A subsequent adherence evaluation (Step S400 or S600) based on the (valid) nebulizing session/operation obtained based on the conditions exemplified above be performed. Thereby it may be determined that nebulizing was according to the therapy protocol.

If in Step S400, the adherence of individual nebulizing operations is evaluated instead, it may be found that the individual nebulizing durations are shorter than the prescribed minimum nebulizing duration. As a result, without the (conditional) clustering, it would erroneously be determined that the nebulizing was not according to the therapy protocol, although it actually was in accordance with the therapy protocol.

On the other hand, if in step S600, the adherence of a nebulizing session is evaluated instead, it may be found that an insufficient number of nebulizing sessions was performed. After all, the number of (valid) nebulizing operations may be in accordance with the therapy protocol. If these (valid) nebulizing operations are however clustered into a nebulizing session, they are erroneously no longer treated as individual nebulizing operations. Because the number of nebulizing sessions may be fewer than the number of nebulizing operations clustered into nebulizing sessions, it may erroneously be determined that nebulizing was not according to the therapy protocol, although it actually was in accordance with the therapy protocol.

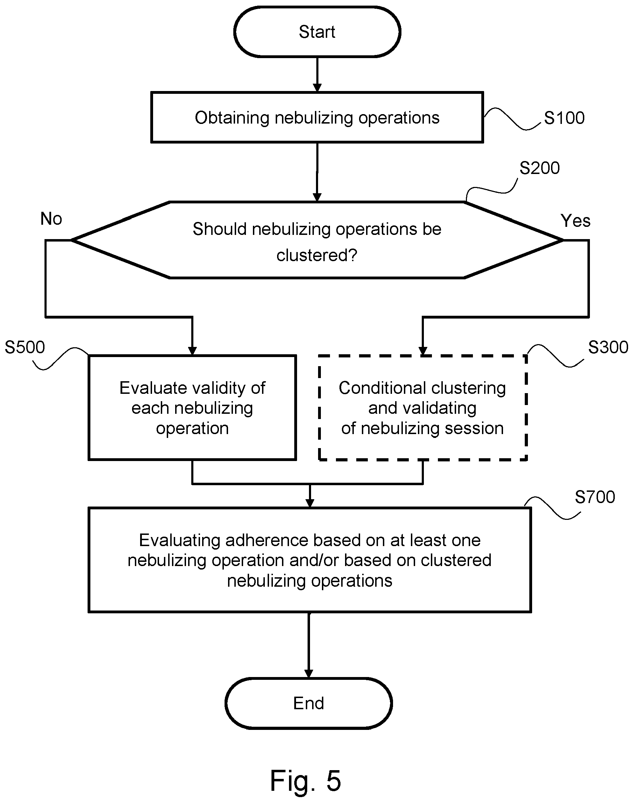

Although the above description in reference to Fig. 4 explained a case where nebulizing operations and nebulizing sessions are evaluated separately in steps S600 and S400, the present invention is not limited thereto. That is to say, if it is determined that one (or more) nebulizing operation is valid and/or one (or more) nebulizing session is valid, then the adherence to the therapy protocol may be evaluated for a combination of the valid nebulizing operation(s) and/or the valid nebulizing session(s) as illustrated in Step S700 shown in Fig. 5. That is to say, the disclosure related to rules for evaluating adherence of a nebulizing operation may mutually be applied/used as rules for evaluating adherence of a nebulizing session and vice versa. However, for conciseness of explaining the present invention, this description preferably refers to the steps of Fig. 4 instead of Fig. 5.

As mentioned above, an aerosol nebulizer 30 may comprise the aerosol generator 31 for nebulizing a liquid medication according to the therapy protocol. It may further comprise a (first) processor, micro-processor, controller, microcontroller or the like 32 for performing the steps of any embodiment of the medical evaluation method. Thereby, data transmitted by the aerosol nebulizer 30 may be limited to valid nebulizing operations/sessions to avoid providing unnecessary data to the subsequent evaluation or analysis processing.

In this regard, the evaluation or analysis processing based on a valid nebulizing session and/or a valid nebulizing operation may comprise evaluating an adherence to the (prescribed) therapy protocol. Specifically, the medical evaluation method may further comprise evaluating (Step S400 and/or Step S600) an adherence to the therapy protocol based on the nebulizing session evaluated as valid and/or the first and/or the second nebulizing operation evaluated as valid. In this context, the evaluation of the adherence to the therapy protocol may generally refer to an evaluation as to whether the user/patient performs an inhalation process (indicated by the nebulizing operations performed by the aerosol nebulizer 30) as prescribed by the therapy protocol and/or as to whether the aerosol nebulizer 30 itself operates as required by the therapy protocol.

This adherence evaluation may be performed at a level of the aerosol nebulizer 30, the control device 30 or the medical evaluation device 10, or a combination thereof. For a concise explanation of a preferred embodiment, the following description makes use of the scenario that the medical evaluation device 10, as shown in Fig. 2, performs the adherence evaluation, but the present invention is not limited thereto and any of the devices mentioned above may be used instead or in combination. That is to say, a preferred embodiment of the medical evaluation system is explained in reference to Fig. 2.

Accordingly, a medical evaluation system may comprise the aerosol nebulizer 30, with a first communication interface 33 for transmitting nebulizing (session/operation) data being based on at least one (valid) nebulizing session and/or nebulizing operation. The system may further comprise the medical evaluation device 10, with a second communication interface 100 for receiving the nebulizing (session/operation) data, and a (second) processor 110 for evaluating the adherence to the therapy protocol based on the nebulizing (session/operation) data. Thereby, no large amounts of memory for storing a history of adherence data needs to be provided by the aerosol nebulizer 30, allowing the aerosol nebulizer 30 to be made as small as possible. Also, the nebulizing (session/operation) data may be packeted data relating to at least one nebulizing session or nebulizing operation. Thereby, nebulizing data may only be provided at the end of the predetermined data recording period described above e.g., to buffer an amount of valid nebulizing operations/sessions and to transmit data associated thereto as a burst or packet transmission. Doing so may reduce the number of transmissions for providing data for the adherence evaluation. Doing so also reduces the workload put on the device performing the adherence evaluation since no invalid nebulizing operations/sessions need to be filtered out before the adherence evaluation can commence.

According to a further preferred embodiment, the daily adherence rate and/or a cumulative adherence rate may be calculated for a defined time period of said therapy protocol (e.g., for a plurality of days). Here, the adherence rate may be calculated based on the following expression (1):

$$
\text{adherence rate} = \frac{((N_{IV}/N_{IT})_{Day\,1} + (N_{IV}/N_{IT})_{Day\,2} + \ldots + (N_{IV}/N_{IT})_{Day\,n})}{n} \quad (1)
$$

In this expression which $N_{IV}$ may be a number of valid inhalations e.g., individual inhalation operations/ clustered nebulizing sessions, when a predefined required minimum inhalation duration is reached for an individual (un-clustered) inhalation operation and/or a clustered nebulizing session, $N_{IT}$ may be a number of inhalations defined according to the prescribed therapy protocol, and n may be the number of days in the defined time period. Here, the number of prescribed daily inhalation sessions/operations and the minimum duration of one valid inhalation may be drug specific, user-specific or study-specific (for a clinical trial). Based on the above expression (1), the cumulative adherence rate refers to a ratio between a sum of daily adherence rates of each therapy day within the selected time period of the therapy protocol and the total days of the selected time period. Based on the above equation (1), the daily adherence rate may be determined by considering the adherence rate for n=1.

The calculated daily adherence rate and/or cumulative adherence rate for the defined time period may be displayed on a display unit 130 of the medical evaluation device 10, as shown in Fig. 2. E.g., the user (may also be clinical trial personnel, a medical doctor, physician or the like) may use a web-interface to access the nebulizing data and the calculated adherence data and view the data e.g., in a table view of all inhalation records, graphical view of all inhalation records, and/or an adherence report (to be described later). Such adherence reports may be stored locally in the medical evaluation device 10 or exported to an external device. Such a report may comprise any of a corresponding time period, a patient identification, a study number, and/or a graphical display of daily/cumulative adherence rate over a defined period of said medical protocol.

According to another embodiment, a computer program is provided that includes instructions which, when executed on one or a plurality of data processors, cause the data processor or the plurality of data processors to implement the medical evaluation device 10 as described above.

The above respective modules/units of the medical evaluation device 10 may thus be implemented by a respective central processing unit (CPU) that may include one or a plurality of processors, a microprocessor or other processing logic that interprets and executes instructions as defined by the computer program and stored in a main memory. The main memory may include a random access memory (RAM), a read only memory (ROM) or other type of dynamic storage device that may store information and instructions for execution by the respective modules/units. The ROM may include a ROM device or another type of static storage device that may store static information and instructions for use by the processing unit.

The each of or a combination of the devices referred to above may perform the steps according to embodiments of the medical evaluation method also shown in Fig. 3 and 4 in response to one or more processing units executing software algorithms contained in a computer-readable medium, such as the main memory, RAM, ROM and/or storage device comprised by the devices. A computer-readable medium may be defined as a physical or a logical memory device. E.g., a logical memory device may include memories within a single physical memory device or distributed across multiple physical memory devices. Each of the main memory, RAM, ROM and storage device may include computer-readable media with instructions as program code. The software algorithms may be read into the main memory for another computer-readable medium, such as a storage device or from another device via the communication interface.

The software algorithms contained in the main memory may cause the processing unit(s) including a data processor, when executed on the processing unit, to perform operations or processes described herein. Alternatively, hard-wired circuitry may be used in place of or in combination with the software algorithms to implement processes and/or operations described herein. Thus, implementations described herein are not limited to any specific combination of hardware and software.

Further, the respective units of the devices referred to above may be implemented in hardware, software, Field Programmable Gate Arrays (FPGAs), application-specific integrated circuits (ASICs), firmware or the like.

It will be apparent to those skilled in the art that various modifications and variations can be made in the devices, entities and methods of the present invention as well as in the construction of this invention without departing from the scope of the invention.

The invention has been described in relation to particular embodiments and examples which are intended in all aspects to be illustrative rather than restrictive. Those skilled in the art will appreciate that many different combinations of hardware, software and/or firmware will be suitable for performing the present invention.

**Claims**

1. A medical evaluation method for evaluating a validity of a nebulizing session prescribed by a therapy protocol when using an aerosol nebulizer (30) comprising an aerosol generator (31) for nebulizing a liquid medication, the method comprising:

   obtaining (S100) nebulizing data indicating a first nebulizing operation and a second nebulizing operation of the aerosol nebulizer, each of the first and second nebulizing operation having an individual nebulizing duration at an individual nebulizing time and a nebulizing status code;
   deciding (S200) whether to perform clustering, based on the individual nebulizing time and/or the nebulizing status code and/or the individual nebulizing duration of the first and/or the second nebulizing operation and/or the liquid medication to be nebulized in the first and/or second nebulizing operation; and
   if it is decided to perform clustering, the method further comprising:

   clustering (S310) the first and second nebulizing operation into the nebulizing session;
   determining (S320) a total nebulizing duration for the nebulizing session based on the individual nebulizing duration of the first and second nebulizing operation clustered into the nebulizing session; and
   evaluating (S330) the validity of the nebulizing session based on a prescribed nebulizing duration specific to the therapy protocol and the total nebulizing duration determined for the nebulizing session, the prescribed nebulizing duration preferably being of more than or equal to four minutes or more than or equal to one minute.

2. The medical evaluation method according to claim 1, if it is decided not to perform clustering, the method further comprising:
   evaluating (S500) a validity of the first and/or the second nebulizing operation based on:

   the prescribed nebulizing duration specific to the therapy protocol and the individual nebulizing duration of the first and/or second nebulizing operation, or
   the prescribed nebulizing duration specific to a shortest possible nebulizing duration of more than one possible nebulizing durations, each prescribed by the therapy protocol for each of more than one liquid medications, and the individual nebulizing duration of the first and/or second nebulizing operation.

3. The medical evaluation method according to claims 1 or 2, further comprising:
   evaluating an adherence to the therapy protocol based on the nebulizing session evaluated as valid and/or the first and/or the second nebulizing operation evaluated as valid.

4. The medical evaluation method according to any one of claims 1 to 3, wherein the first and second nebulizing operation are clustered into the nebulizing session if the individual nebulizing time of each of the first and second nebulizing operation lies within a predetermined allowable nebulizing session time window.

5. The medical evaluation method according to claim 4,
   wherein the allowable nebulizing time window is specific to the liquid medication, and preferably is of a duration of up to two hours.

6. The medical evaluation method according to claim 4 or 5, wherein the allowable nebulizing time window is based on a predetermined minimum in vivo concentration.

7. The medical evaluation method according to any one of claims 1 to 6, wherein the first and second nebulizing operation are clustered into the nebulizing session if an inactivity period between the first and second nebulizing operation is less than or equal to a predetermined inactivity period.

8. The medical evaluation method according to claim 7,
wherein the predetermined inactivity period is specific to the liquid medication, and preferably is of a duration of less than or equal to thirty minutes.

9. The medical evaluation method according to any one of claims 1 to 8, wherein the first and second nebulizing operation is associated with an inhalation process and/or with operating the aerosol generator of the aerosol nebulizer.

10. The medical evaluation method according to any one of claims 1 to 10, wherein the evaluating (S330, S500) the validity is based on a timely distance between two nebulizing sessions, between two nebulizing operations or between a nebulizing session and a nebulizing operation.

11. The medical evaluation method according to any one of claims 1 to 10, wherein the therapy protocol specifies at least two nebulizing sessions as having to take place within a predetermined therapy period assigned to each of the at least two nebulizing sessions or operations.

12. The medical evaluation method according to claim 11,
wherein the first and second nebulizing operation are clustered into a first nebulizing session of the at least two nebulizing sessions if the individual nebulizing time of each of the first and second nebulizing operation is before a therapy period of a second nebulizing session of the at least two nebulizing sessions.

13. The medical evaluation method according to claim 11 or 12, wherein the first and second nebulizing operation are clustered into a second nebulizing session of the two or more nebulizing sessions if the individual nebulizing time of each of the first and second nebulizing operation is after a therapy period of a first nebulizing session and after or during a therapy period of the second nebulizing session.

14. The medical evaluation method according to claim 12 or 13, wherein the first nebulizing session is before the second nebulizing session.

15. The medical evaluation method according to any one of claims 1 to 14, wherein the individual nebulizing time is a time at which nebulizing begins or ends.

16. The medical evaluation method according to any one of claims 1 to 15, wherein the nebulizing status code indicates whether: a nebulizing operation has finished as expected; the aerosol nebulizer has lost power; a nebulizing operation has been started without providing the liquid medication; the aerosol nebulizer has been manually shutdown; and/or a timeout has been reached and/or a failure mode has stopped nebulization.

17. The medical evaluation method according to any one of claims 1 to 16, wherein the deciding (S200) comprises not to perform clustering if the individual nebulizing duration of the first and/or second nebulizing operation is shorter than a predetermined minimum nebulizing duration, the minimum nebulizing duration preferably being 10 seconds or less, or 15 seconds or less.

18. The medical evaluation method according to any one of claims 1 to 17, wherein the evaluating (S330, S500) the validity comprises evaluating of the individual nebulizing duration and/or the total nebulizing duration is longer than a predetermined minimum nebulizing duration.

19. The medical evaluation method according to claim 18, wherein the deciding (S110) comprises deciding not to perform clustering if the nebulizing status code of the first and/or second nebulizing operation or the duration of both nebulization indicates that a nebulizing operation has finished as expected.

20. The medical evaluation method according to any one of claims 1 to 19, wherein the deciding (S110) comprises not to perform clustering if nebulizing status code of the first and/or second nebulizing operation indicates that a timeout has been reached, preferably when the aerosol nebulizer is in an inhalation mode, a pause mode and/or a maintenance mode.

21. The medical evaluation method according to any one of claims 1 to 20 further comprising:

   providing an enquiry notification of whether the first and/or second nebulizing operation is to be clustered into the nebulizing session with the second and/or first nebulizing operation,
   wherein the first and second nebulizing operation are clustered into the nebulizing session if the enquiry notification is confirmed.

22. The medical evaluation method according to any one of claims 1 to 21, further wherein

   the nebulizing data indicates plurality of nebulizing operations including the first and second nebulizing operation and, preferably, a third nebulizing operation and/or a fourth nebulizing operation of the aerosol nebulizer, each of the plurality of nebulizing operations having an individual nebulizing duration at an individual nebulizing time and a nebulizing status code;
   the method further comprising:
   deciding (S200) whether to perform clustering, based on the individual nebulizing time and/or the nebulizing status code and/or the individual nebulizing duration of at least one of the plurality nebulizing operations and/or the liquid medication to be nebulized in the at least one of the plurality of nebulizing operations; and
   if it is decided to perform clustering, the method further comprising:

      clustering (S310) at least two of the plurality of nebulizing operations into the nebulizing session;
      determining (S320) a total nebulizing duration for the nebulizing session based on the individual nebulizing duration of the at least two of the plurality of nebulizing operations clustered into the nebulizing session; and
      evaluating (S330) the validity of the nebulizing session based on a prescribed nebulizing duration specific to the therapy protocol and the total nebulizing duration determined for the nebulizing session.

23. An aerosol nebulizer comprising:

   an aerosol generator for nebulizing a liquid medication according to a therapy protocol; and
   a processor for performing the steps according to any one of claims 1 to 22.

24. A medical evaluation system comprising:

   an aerosol nebulizer according to claim 23, and further comprising
   a first communication interface for transmitting nebulizing session data being based on at least one nebulizing session; and
   a medical evaluation device comprising:

      a second communication interface for receiving the nebulizing session data; and
      a second processor for evaluating an adherence to a therapy protocol based on the nebulizing session data.

25. The medical evaluation system according to claim 24, wherein the nebulizing session data is packeted data relating to the at least one nebulizing session or one nebulizing operation.

Fig. 1

Fig. 2

Start

Obtaining nebulizing operations — S100

Should nebulizing operations be clustered? — S200

No     Yes

S300

Clustering nebulizing operations into nebulizing session — S310

Determine total nebulizing duration of nebulizing session — S320

Validating nebulizing session based on total nebulizing duration and time of nebulizing — S330

End

Fig. 3

```
                    ┌─────────────┐
                    │    Start    │
                    └──────┬──────┘
                           │
                           ▼
        ┌──────────────────────────────────────┐
        │    Obtaining nebulizing operations    │         S100
        └──────────────────┬───────────────────┘
                           │                              S200
                           ▼
  No      ╱───────────────────────────────────╲      Yes
  ┌──────<    Should nebulizing operations be    >──────┐
  │        ╲            clustered?              ╱        │
  │         ╲───────────────────────────────────╱       │
  │                                                     │
  ▼                                                     ▼
S500                                                   S300
┌──────────────────┐                    ┌ ─ ─ ─ ─ ─ ─ ─ ─ ─ ┐
│  Evaluate validity│                      Conditional clustering
│  of each nebulizing│                   │  and validating    │
│     operation     │                       of nebulizing session
└─────────┬─────────┘                    └ ─ ─ ─ ─ ┬ ─ ─ ─ ─ ┘
          │                                        │
S600      ▼                             S400        ▼
┌──────────────────┐                    ┌──────────────────┐
│ Evaluating adherence│                  │ Evaluating adherence│
│ based on at least one│                 │ based on clustered  │
│ nebulizing operation │                 │ nebulizing operations│
└─────────┬─────────┘                    └─────────┬─────────┘
          │                                        │
          │          ┌─────────────┐               │
          └─────────▶│     End     │◀──────────────┘
                     └─────────────┘
```

Fig. 4

```
                      ┌──────────────┐
                      │    Start     │
                      └──────┬───────┘
                             │
                             ▼
        ┌────────────────────────────────────────┐
        │    Obtaining nebulizing operations      │ ─── S100
        └────────────────────┬───────────────────┘
                             │                         S200
                             ▼
   No  ╱────────────────────────────────────────╲  Yes
      ╱      Should nebulizing operations be       ╲
      ╲              clustered?                     ╱
       ╲────────────────────────────────────────  ╱
   │                                                │
   │                                                │
   ▼                                                ▼
 S500                                             S300
┌──────────────────┐          ┌─ ─ ─ ─ ─ ─ ─ ─ ─ ─┐
│  Evaluate validity│          │  Conditional       │
│   of each         │          │  clustering        │
│   nebulizing      │          │  and validating    │
│   operation       │          │  of nebulizing     │
│                   │          │  session           │
└─────────┬─────────┘          └─ ─ ─ ─ ┬─ ─ ─ ─ ─ ┘
          │                             │
          └──────────────┬──────────────┘
                         ▼                        S700
        ┌────────────────────────────────────────┐
        │  Evaluating adherence based on at least │
        │  one nebulizing operation and/or based  │
        │  on clustered nebulizing operations     │
        └────────────────────┬───────────────────┘
                             │
                             ▼
                      ┌──────────────┐
                      │     End      │
                      └──────────────┘
```

## Fig. 5

| Code | Explanation | Valid nebulizing / complete dose |
|---|---|---|
| 1 | Loss of supply voltage to aerosol nebulizer | No |
| 2 | Disconnection of aerosol nebulizer from control device | No |
| 3 | Nebulizing started without liquid medication in the aerosol nebulizer | No |
| 4 | Nebulizing / Inhalation finished as expected / prescribed by therapy protocol | Yes |
| 5 | Manual shutdown of aerosol nebulizer | No |
| 6 | Battery / Power supply empty | No |
| 7 | Timeout during inhalation mode (e.g., triggered at ≥ 1200 s) | No |
| 8 | Timeout during pause mode (e.g., triggered at > 600 s) | No |
| ... | ... | ... |
| 101-108 | Timeout during maintenance mode (e.g., triggered at > 600 s) | No |

Fig. 6

Europäisches
Patentamt

European
Patent Office

Office européen
des brevets

# EUROPEAN SEARCH REPORT

Application Number

EP 23 15 4428

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/038854 A1 (FUCHS CAROLA [DE] ET AL) 7 February 2019 (2019-02-07) | 1-3,9, 18,23-25 | INV.<br>G16H20/10 |
| A | * paragraphs [0016], [0043], [0044], [0045]; figure 2 * | 4-8, 10-17, 19-22 | G16H40/63<br>G16H40/67 |

-----

TECHNICAL FIELDS
SEARCHED      (IPC)

G16H

The present search report has been drawn up for all claims

| Place of search | Date of completion of the search | Examiner |
|---|---|---|
| Munich | 3 July 2023 | Samulowitz, Michael |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 23 15 4428

This annex lists the patent family members relating to the patent documents cited in the above-mentioned European search report.
The members are as contained in the European Patent Office EDP file on
The European Patent Office is in no way liable for these particulars which are merely given for the purpose of information.

03-07-2023

| Patent document cited in search report | | Publication date | Patent family member(s) | | Publication date |
|---|---|---|---|---|---|
| US 2019038854 | A1 | 07-02-2019 | CA | 3012575 A1 | 17-08-2017 |
| | | | EP | 3203397 A1 | 09-08-2017 |
| | | | EP | 3414687 A1 | 19-12-2018 |
| | | | ES | 2786183 T3 | 09-10-2020 |
| | | | JP | 2019504696 A | 21-02-2019 |
| | | | JP | 2023062005 A | 02-05-2023 |
| | | | US | 2019038854 A1 | 07-02-2019 |
| | | | WO | 2017137424 A1 | 17-08-2017 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 10243371 A1
- US 20060237001 A1
- WO 2017137424 A1